Europäisches Patentamt

European Patent Office

Office européen des brevets

(11) Publication number: **0 511 454 A1**

# EUROPEAN PATENT APPLICATION

(21) Application number: **91830177.1**

(22) Date of filing: **30.04.91**

(51) Int. Cl.⁵: **A61K 7/06**

(43) Date of publication of application:
**04.11.92 Bulletin 92/45**

(84) Designated Contracting States:
**AT BE CH DE DK ES FR GB GR IT LI LU NL SE**

(71) Applicant: **Fiorentini, Paolo**
**Via Bassi 12**
**San Vincenzo di Galliera (Bologna)(IT)**
Applicant: **Bombarda, Feliciana**
**Via Bassi 12**
**San Vincenzo di Galliera (Bologna)(IT)**

(72) Inventor: **Fiorentini, Paolo**
**Via Bassi 12**
**San Vincenzo di Galliera (Bologna)(IT)**
Inventor: **Bombarda, Feliciana**
**Via Bassi 12**
**San Vincenzo di Galliera (Bologna)(IT)**

(74) Representative: **Sassatelli, Franco T., Dr.**
**c/o INIP via Ruggi 5**
**I-40137 Bologna(IT)**

(54) **Hair polyvalent adjuvant containing capsicum extract.**

(57) The invention enables by means of the capsicum extract (5) a vasodilator function, which by means of the increased blood circulation performs a vitalizing oxygenation of the hair-bulb thus setting itself against its loss through asphysia, while by means of the essential lavender oil (7) it enables an antiseptic function which destroys the hair-bulb parasites as well as with a bio-stimulant function.

The invention refers to an hair-bulb strengthening lotion using the placenta extract synergic action to enable a simultaneous intervention with the other composition elements. The invention starts from the assumption that in the adjuvant preparation determining for the hair-fall prevention it is advisable to value and prevent many of those factors which are responsible of the hair-fall itself. This, since the experience has proved that in most part of the subjects, the hair-fall cause is the different elements sum which affect the whole hair and dermis morphology. In connection the excipients have been singled out so to determine the least possible interference with the active principles, whereas the preservatives, beside not interfering with the active principles, have been inserted in way to be effective in the less dose possible so to obtain the widest action spectrum and the major dermis tollerance possible.

Principally the adjuvant permits by means of the capsicum extract (5) presence a vasodilator function, which by means of the increased blood circulation performs a vitalising oxygenation of the hair-bulb thus setting itself against its loss through asphyxia, while by means of the essential lavender oil (7) it enables an antiseptic function which destroys the hair-bulb parasites and it enables a biostimulant function. As a result is obtained an hair treatment polyvalent adjuvant which permits new kind results combine with the use of the already known bovine placenta extract properties in the body hair treatment.

The lotion base composition according the invention obtained by elements cold mixing, stated in their weight in grams percentage, is given by: 1) distilled water 93,495. 2) polyvinyl pyrrolidone 0,5. 3) cow placenta extract 1,00; used for its known stimolating and regulating properties in the damaged tissues growth. 4) calcium pantothenate 0,8; consisting of a vegetable extracts mixture and "B" group vitamins in which the presence between the vegetable of the nettle allows to the product a soft vasodilator and stimulating action of the piliferous function besides the known pantothenic acid property. 5) capsicum extract 0,8; consisting of vegetable extracts mixture with organic sulfur base and recommended in the dandruff treatment and in the scalp treatment with excedent sebaceous secretion. 6) propylene glycol monomethyl ether 2,55; a plasticizer which laid as a protective film around the hair protects it against the atmosphere agents and against the air suspended ones. 7) essential lavender oil 0,05; said element is hot solubilized and perfumes the dermis producing a bio-stimulant action. 8) essential oil mixture 0,35; said element is hot solubilized, with nonyl phenol - 10 moly. With the above said elements are moreover added other elements giving a preservative action to the product such: 9) methyl chloroisothiazolinone and methyl isothianzolinone 0,25; antibacterial agent with wide action spectrum. 10) methyl ester 4-hydroxybenzoic acid 0,175, an antifermentation. 11) parapropil 0,03; an antioxidant which avoids the product decay.

Essential element of the adjuvant is the capsicum extract (5) which analized consists of the following standard: denomination IIIC, water, monoethylether diethylene glycol; monifenil (10) OE; propylene glycol, nettle and pepper extracts; soluble sulfur; mesulfene, resorcinol (0,4%), birch tar essential oil, camphor essential oil, Bay St. Thomas essential oil, caramel E150. It appears also as liquid and transparent, with green-brown colour, a density at 20° of 1.020-1.031 and an index of refraction at 20° of 1.391-1.405.

The present adjuvant may be produced in ampules and it may be used in conventional way with a weekly frequence by means of frictions.

**Claims**

1. Hair polyvalent adjuvant, characterized by the fact that the capsicum extract (5) presence permits a vasodilator function, which by means of the increased blood circulation performs a vitalizing oxygenation of the hair-bulb thus setting itself against its loss through asphyxia, while by means of the essential lavender oil (7) it enables an antiseptic function which destroys the hair-bulb parasites and it enables a biostimulant function.

2. Hair polyvalent adjuvant, as in claim 1), characterized by the fact that the lotion base composition, obtained by elements cold mixing, is given by: distilled water 93,495; polyvinyl pyrrolidone 0,5; cow placenta extract 1,00; calcium pantothenate 0,8; capsicum extract 0,8; propylene glycol monomethyl ether 2,55; essential lavender oil 0,05; essential oil mixture 0,35; methyl chloroisothiazolinone and methyl isothiazolinone 0,25; methyl ester 4-hydroxybenzoic acid 0,175 and para-propil 0,03.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | FR-A-2588756 (ABRAMOWICZ S.)<br>* page 2, lines 22 - 26 *<br>--- | 1-2 | A61K7/06 |
| Y | US-A-3824304 (VILLANUEVA A.F.)<br>* claim 1 *<br>--- | 1-2 | |
| Y | GB-A-1458349 (SYKORA F.)<br>* page 1, column 1, lines 87 - 90 *<br>* page 2, column 1,2, lines 1 - 23 *<br>--- | 1-2 | |
| Y | GB-A-856914 (MARCHETTI S.)<br>* page 1, column 2, lines 64 - 66 *<br>--- | 1-2 | |
| Y | EP-A-394920 (REVLON)<br>* page 4; claims 1-5 *<br>* page 4; example *<br>--- | 1-2 | |
| A | DE-A-2604201 (MESCH CASA COSMETICA)<br>* page 10, lines 5 - 7 *<br>----- | 2 | |
| | | | TECHNICAL FIELDS SEARCHED (Int. Cl.5 )<br><br>A61K |

The present search report has been drawn up for all claims

| Place of search | Date of completion of the search | Examiner |
|---|---|---|
| BERLIN | 03 DECEMBER 1991 | AVEDIKIAN P.F. |

EPO FORM 1503 03.82 (P0401)